# EUROPEAN PATENT APPLICATION

(11) **EP 4 289 954 A1**
(43) Date of publication of application: **13.12.2023**
(21) Application number: 22749288.1
(22) Date of filing: 07.04.2022
(51) Int. Cl.: C12N 15/113, A61K 48/00, A61P 27/06

(54) **SGRNA TARGETING AQP1 MRNA, AND VECTOR AND USE THEREOF**

(30) Priority: 07.02.2021 CN 202110174416
(71) Applicant: Guangzhou Reforgene Medicine Co., Ltd., Guangzhou, Guangdong 510700 (CN)
(72) Inventor: LIANG, Junbin, Guangzhou, Guangdong 510700 (CN); ZHANG, Kechuang, Guangzhou, Guangdong 510700 (CN); XU, Hui, Guangzhou, Guangdong 510700 (CN)
(74) Representative: Dehns
(86) International application number: PCT/CN2022/085442
(87) International publication number: WO 2022/167009

(57) **Abstract**

Provided are an sgRNA targeting Aqpl mRNA, and a vector and use thereof. The sgRNA comprises a targeting structure and a backbone sequence, the targeting structure being complementary to a target nucleic acid Aqpl mRNA sequence, the backbone sequence being capable of binding to or interacting with a Cas protein. After the vector is used to deliver the sgRNA to cells, Aqpl mRNA is targeted and the Aqpl mRNA level is reduced, so as to treat diseases in which downregulation of Aqpl expression is beneficial, including but not limited to diseases related to Aqpl overexpression, including cancer and glaucoma.

## Description

### TECHNICAL FIELD

The present disclosure relates to the technical field of biomedicine, and especially an sgRNA targeting Aqpl mRNA, and a vector and use thereof.

### BACKGROUND

Aquaporin 1 (Aqpl) is a class of transmembrane proteins widely distributed *in vivo.* Aqpl is mainly involved in the regulation of water transport *in vivo.* Besides, it also plays an important role in angiogenesis, cell migration and cell growth. MeiraGTx Company has developed a gene therapy product, AAV-Aqp1. Radiation-Induced Xerostomia (RIX) is treated by overexpressing Aqpl gene in injured salivary glands by AAV vector. MeiraGTx Company is currently conducting phase I/II clinical trials of AAV-Aqp1 gene medicine. In addition, Aqpl was found to be related to the pathogenesis of glaucoma.

Glaucoma is a neurodegenerative disease characterized pathologically by apoptosis of retinal ganglion cell (RGC) and degeneration of axons. The apoptosis of ganglion cells is mainly related to intraocular pressure (IOP). The IOP is mainly maintained by aqueous humor. When the ciliary body (CB), the generating tissue of the aqueous humor, and the trabecular meshwork (TM), the main discharge tissue of the aqueous humor, fail, the circulation system of aqueous humor may malfunction, eventually causing IOP to rise, compressing RGCs, and eventually leading to irreversible loss of visual field. Functional loss of Aqp1 reduces aqueous humor production and lowers intraocular pressure. At present, lowering intraocular pressure is still a routine means of treating glaucoma.

Since the visual field damage caused by glaucoma is irreversible, the main role of current drugs for glaucoma is to reduce intraocular pressure (IOP). There are currently five main classes of IOP-lowering drugs: α-adrenergic agonists, β-adrenergic antagonists, cholinergic agonists, prostaglandins, and carbonic anhydrase inhibitors. They are mainly administered in the form of eye drops, which requires frequent administration and has certain side effects. Suzhou Ribo Life Science Co., Ltd. has developed an innovative siRNA (small interfering RNA) nucleic acid drug QPI-1007 for optic nerve protection, which is currently in the global critical research of phase II/III clinical trial. The drug is designed to temporarily inhibit the expression of the pro-apoptotic protein caspase 2. The drug also prevents the apoptosis of retinal ganglion cells (RGC), thereby reducing visual field damages.

Studies have found that reducing the expression of Aqp1 can also reduce angiogenesis and decelerate tumor progression (Nico et al, Cancer Letters, 2010, 294(2):135-138).

CRISPR/Cas (clustered regularly interspaced short palindromic repeats/CRISPR-associated proteins) system is currently the most widely used gene editing technology. CRISPR/Cas system has been used to edit the Aqpl gene in the prior art. Moreover, the prior art targets the exon region of the Aqp1 gene and cuts genomic DNA, resulting in mutation of the original Aqp1 gene.

### CONTENT OF THE PRESENT INVENTION

The purpose of the present disclosure is to provide an sgRNA (single guide RNA, single molecule gRNA) that can target human Aqpl mRNA, and a vector and use thereof.

In a first aspect, the present disclosure claims an sgRNA.

The sgRNA claimed in the present disclosure can target human Aqp1 mRNA.

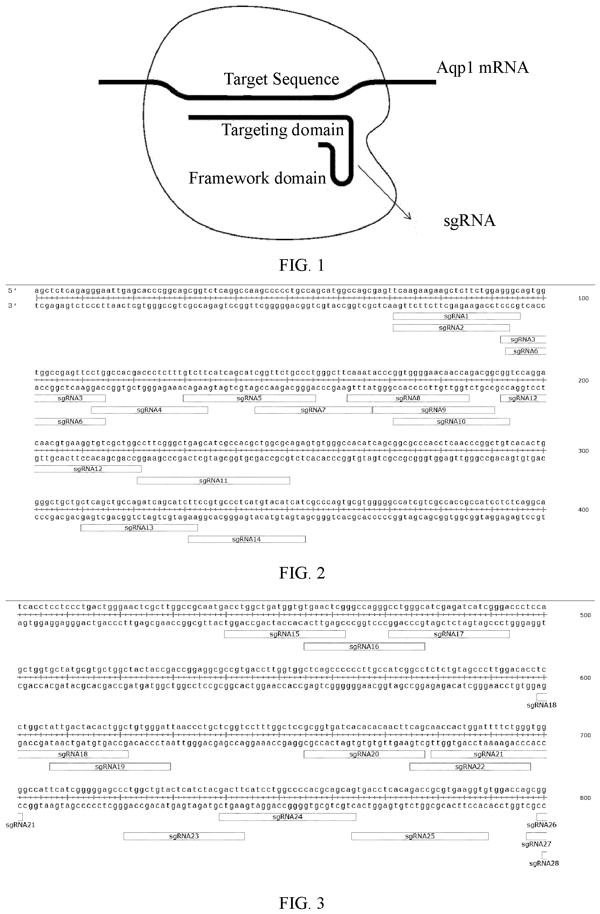

The sgRNA includes a targeting domain (guide sequence) and a framework domain (framework sequence), as shown in FIG. 1. The targeting domain is complementary (completely complementary or partially complementary) to a target nucleic acid Aqpl mRNA sequence, and the framework sequence can bind to or interact with a Cas protein. Those skilled in the art can link the guide sequence to 5' end or 3' end of the framework sequence as required.

Herein, the Cas protein is preferably a Cas13 protein. The Cas13 protein is Cas13a, Cas13b, Cas13c, Cas13d, Cas13e or Cas13f protein.

In some examples, the targeting domain has a sequence comprising any one of sequences shown in SEQ ID NO: 1 to SEQ ID NO: 31.

In some examples, the targeting domain is a sequence selected from any one of SEQ ID NO: 1 to SEQ ID NO: 31.

In some examples, the targeting domain of the sgRNA has a sequence comprising any one of sequences shown in SEQ ID NO: 1, SEQ ID NO: 2, SEQ ID NO: 4, SEQ ID NO: 5, SEQ ID NO: 7, SEQ ID No: 8, SEQ ID NO: 9, SEQ ID NO: 10, SEQ ID NO: 12, SEQ ID NO: 14, SEQ ID NO: 15, SEQ ID NO: 17, SEQ ID NO: 18, SEQ ID NO: 20, SEQ ID NO: 21, SEQ ID NO: 23, SEQ ID NO: 24, SEQ ID NO: 26, SEQ ID NO: 28, SEQ ID NO: 30, and SEQ ID NO: 31.

In some examples, the targeting domain of the sgRNAis a sequence selected from any one of SEQ ID NO: 1, SEQ ID NO: 2, SEQ ID NO: 4, SEQ ID NO: 5, SEQ ID NO: 7, SEQ ID NO: 8, SEQ ID NO: 9, SEQ ID NO: 10, SEQ ID NO: 12, SEQ ID NO: 14, SEQ ID NO: 15, SEQ ID NO: 17, SEQ ID NO: 18, SEQ ID NO: 20, SEQ ID NO: 21, SEQ ID NO: 23, SEQ ID NO: 24, SEQ ID NO: 26, SEQ ID NO: 28, SEQ ID NO: 30, and SEQ ID NO: 31.

In some examples, the targeting domain of the sgRNAis a sequence selected from any one of SEQ ID NO: 2, SEQ ID NO: 5, SEQ ID NO: 21, SEQ ID NO: 24, and SEQ ID NO: 31.

The framework sequence of the sgRNA of the present disclosure can be designed in a conventional manner.

The framework sequence can be selected differently according to different Cas proteins, which can be easily realized by those skilled in the art. In some examples, the sgRNA uses a gRNA framework sequence corresponding to Cas13a, Cas13b, Cas13c, Cas13d, Cas13e or Cas13f.

In some examples, the sgRNA is used in combination with a Cas13 nuclease or a nucleic acid molecule encoding a Cas13 nuclease. In some examples, the sgRNA is used in combination with the Cas13 nuclease. In some examples, the Cas13 nuclease is Cas13a, Cas13b, Cas13c, Cas13d, Cas13e or Cas13f.

The Cas13 nuclease includes natural Cas13 protein, artificially modified Cas13 protein, Cas13 mutant with nuclease activity loss or reduction (such as a mutation in HEPN domain, such as a mutation in RxxxxH motif), conjugate of Cas13 (non-limiting examples such as conjugate with nuclear localization signal NLS or nuclear export signal NES), fusion protein of Cas13 (non-limiting examples such as a single base editor formed by fusion with deaminase).

In some examples, the sgRNAis used in combination with a Cas13a nuclease, and the framework sequence of the sgRNA is located at 5' end of the guide sequence.

In some examples, the sgRNAis used in combination with a Cas13b nuclease, and the framework sequence of the sgRNA is located at 3' end of the guide sequence.

In some examples, the sgRNAis used in combination with a Cas13c nuclease, and the framework sequence of the sgRNA is located at 5' end of the guide sequence.

In some examples, the sgRNAis used in combination with a Cas13d nuclease, and the framework sequence of the sgRNA is located at 5' end of the guide sequence.

In some examples, the Cas13d nuclease is CasRx.

In some examples, the sgRNA can bind to or interact with Cas13 nuclease. That is, the framework sequence of the sgRNA binds to or interacts with the Cas13 nuclease.

In some examples, the sgRNA may form a complex with Cas13 nuclease.

In some examples, the sgRNA may guide Cas13 nuclease to a target nucleic acid Aqp1 mRNA. That is, the Cas13 nuclease is guided to a target sequence of the target nucleic acid Aqpl mRNA through complementation (complete complementation or partial complementation) between the targeting domain of the sgRNA and the target nucleic acid Aqp1 mRNA sequence.

In some examples, the sgRNA may guide Cas13 nuclease to target nucleic acid Aqp1 mRNA, and target or modify the target nucleic acid Aqp1 mRNA.

In some examples, the sgRNA may form a complex with Cas13 nuclease, and the complex targets or modifies the target nucleic acid Aqpl mRNA.

In some examples, targeting the target nucleic acid Aqp1 mRNA is selected from one or more of the following:
cleaving Aqpl mRNA, visualizing or detecting Aqpl mRNA, labeling Aqpl mRNA, transporting Aqpl mRNA, masking Aqpl mRNA, increasing transcription and/or translation level of Aqp1 mRNA, and decreasing the transcription and/or translation level of Aqp1 mRNA.

In some examples, modifying the target nucleic acid is selected from one or more of the following:
base substitution of Aqpl mRNA, base deletion of Aqpl mRNA, base insertion of Aqpl mRNA, methylation of Aqpl mRNA, demethylation of Aqpl mRNA, and deamination of Aqp1 mRNA.

The sgRNA of the present disclosure can be appropriately chemically modified on any nucleotide.

In a second aspect, the present disclosure claims an sgRNA combination.

The sgRNA combination claimed in the present disclosure may be composed of any two different sgRNAs from the first aspect.

Herein, sequences of the targeting domains of the "any two different sgRNAs from the first aspect" are selected from any two of SEQ ID NO: 1 to SEQ ID NO: 31.

Preferably, in some examples, the sequences of the targeting domains of the "any two different sgRNAs from the first aspect" are selected from any two of SEQ ID NO: 1, SEQ ID NO: 2, SEQ ID NO: 4, SEQ ID NO: 5, SEQ ID NO: 7, SEQ ID NO: 8, SEQ ID NO: 9, SEQ ID NO: 10, SEQ ID NO: 12, SEQ ID NO: 14, SEQ ID NO: 15, SEQ ID NO: 17, SEQ ID NO: 18, SEQ ID NO: 20, SEQ ID NO: 21, SEQ ID NO: 23, SEQ ID NO: 24, SEQ ID NO: 26, SEQ ID NO: 28, SEQ ID NO: 30, and SEQ ID NO: 31.

More preferably, in some examples, the sgRNA combination is selected from any one of the following:
P1. the sgRNA of which the targeting domain has a sequence of SEQ ID NO: 2, and the sgRNA of which the targeting domain has a sequence of SEQ ID NO: 5;
P2. the sgRNA of which the targeting domain has a sequence of SEQ ID NO: 2, and the sgRNA of which the targeting domain has a sequence of SEQ ID NO: 21;
P3. the sgRNA of which the targeting domain has a sequence of SEQ ID NO: 2, and the sgRNA of which the targeting domain has a sequence of SEQ ID NO: 24;
P4. the sgRNA of which the targeting domain has a sequence of SEQ ID NO: 2, and the sgRNA of which the targeting domain has a sequence of SEQ ID NO: 31;
P5. the sgRNA of which the targeting domain has a sequence of SEQ ID NO: 5, and the sgRNA of which the targeting domain has a sequence of SEQ ID NO: 21;
P6. the sgRNA of which the targeting domain has a sequence of SEQ ID NO: 5, and the sgRNA of which the targeting domain has a sequence of SEQ ID NO: 24;
P7. the sgRNA of which the targeting domain has a sequence of SEQ ID NO: 5, and the sgRNA of which the targeting domain has a sequence of SEQ ID NO: 31;
P8. the sgRNA of which the targeting domain has a sequence of SEQ ID NO: 21, and the sgRNA of which the targeting domain has a sequence of SEQ ID NO: 24;
P9. the sgRNA of which the targeting domain has a sequence of SEQ ID NO: 21, and the sgRNA of which the targeting domain has a sequence of SEQ ID NO: 31;
P10. the sgRNA of which the targeting domain has a sequence of SEQ ID NO: 24, and the sgRNA of which the targeting domain has a sequence of SEQ ID NO: 31.

In a third aspect, the present disclosure claims a DNA molecule encoding the sgRNA described in the first aspect above or encoding the sgRNA combination described in the second aspect above.

In a fourth aspect, the present disclosure claims an expression cassette, an expression vector, a recombinant bacterium, a recombinant virus or a transgenic cell line containing the DNA molecule described in the third aspect above.

In some examples, the expression vector comprises two or more nucleotide sequences encoding the sgRNA described in the first aspect.

The above expression vector may express sgRNA for targeting Aqpl mRNA, and it can be understood that those skilled in the art can refer to conventional techniques for construction of the expression vector.

In some examples, the expression vector further comprises a nucleotide sequence encoding Cas13 nuclease. The nucleotide sequence is DNA or RNA. In some examples, the nucleotide sequence encoding the sgRNA is operably linked to a promoter. In some examples, the nucleotide sequence encoding Cas13 nuclease is operably linked to a promoter. The expression vector may express sgRNA and Cas13 nuclease for targeting Aqpl mRNA, and it can be understood that those skilled in the art can refer to conventional techniques for construction of the expression vector.

The nucleotide sequence encoding the sgRNA and the nucleotide sequence encoding the Cas13 nuclease may be located on the same expression vector, or may be located on different expression vectors.

In a fifth aspect, the present disclosure claims a kit.

The kit claimed in the present disclosure may include any one of the following:
i. a Cas protein, and the sgRNA described in the first aspect above;
ii. an expression vector 1 comprising a nucleotide sequence encoding a Cas protein, and the sgRNA described in the first aspect above;
iii. a Cas protein, and an expression vector 2 comprising a nucleotide sequence encoding the sgRNA described in the first aspect above;
iv. the expression vector 1 and the expression vector 2;
v. an expression vector 3 comprising a nucleotide sequence encoding a Cas protein and a nucleotide sequence encoding the sgRNA described in the first aspect above;
vi. a Cas protein, and the sgRNA combination described in the second aspect above;
vii. the expression vector 1, and the sgRNA combination described in the second aspect above;
viii. a Cas protein and an expression vector 4; the expression vector 4 comprising a nucleotide sequence encoding both two sgRNAs in the sgRNA combination described in the second aspect above;
ix. a Cas protein, an expression vector 5 and an expression vector 6; the expression vector 5 comprising a nucleotide sequence encoding any one sgRNA in the sgRNA combination described in the second aspect above; the expression vector 6 comprising a nucleotide sequence encoding the other sgRNA in the sgRNA combination described in the second aspect above;
x. the expression vector 1 and the expression vector 4;
xi. the expression vector 1, the expression vector 5 and the expression vector 6;
xii. an expression vector 7 comprising a nucleotide sequence encoding a Cas protein and a nucleotide sequence encoding both two sgRNAs in the sgRNA combination described in the second aspect above;
xiii. an expression vector 8 and an expression vector 9; the expression vector 8 comprising a nucleotide sequence encoding a Cas protein and a nucleotide sequence encoding any one sgRNA in the sgRNA combination described in the second aspect above; the expression vector 9 comprising a nucleotide sequence encoding the other sgRNA in the sgRNA combination described in the second aspect above;

The Cas protein can bind to or interact with the sgRNA.

In some examples, the Cas protein is a Cas13 protein.

In some examples, the Cas protein is Cas13a, Cas13b, Cas13c, Cas13d, Cas13e or Cas13f.

In some examples, the kit includes any one of the following:
i. a Cas13 protein, and the sgRNA of which the sequence of the targeting domain described above is SEQ ID NO: 5, SEQ ID NO: 21, SEQ ID NO: 24 or SEQ ID NO: 31;
ii. an expression vector 1 comprising a nucleotide sequence encoding a Cas13 protein, and the sgRNA of which the sequence of the targeting domain described above is SEQ ID NO: 5, SEQ ID NO: 21, SEQ ID NO: 24 or SEQ ID NO: 31;
iii. a Cas13 protein, and an expression vector 2 comprising a nucleotide sequence encoding the sgRNA of which the sequence of the targeting domain described above is SEQ ID NO: 5, SEQ ID NO: 21, SEQ ID NO: 24 or SEQ ID NO: 31;
iv. the expression vector 1 and the expression vector 2;
v. an expression vector 3 comprising the nucleotide sequence encoding the Cas13 protein and the nucleotide sequence encoding the sgRNA of which the sequence of the targeting domain described above is SEQ ID NO: 5, SEQ ID NO: 21, SEQ ID NO: 24 or SEQ ID NO: 31;
the Cas13 protein can bind to or interact with the sgRNA.

In some examples, the nucleotide sequence encoding the sgRNA and/or the nucleotide sequence encoding the Cas protein in the expression vector of the fifth aspect is operably linked to a promoter. The expression vector may express sgRNA and/or Cas protein for targeting Aqp1 mRNA in cells (including but not limited to human cells).

The kit may be used to significantly down-regulate expression of Aqpl.

In a sixth aspect, the present disclosure claims a composition.

The composition claimed in the present disclosure may be selected from any one of the following:
I. a composition, comprising: a Cas protein, and the sgRNA described in the first aspect above;
II. a composition, comprising: a nucleic acid molecule 1 encoding a Cas protein, and the sgRNA described in the first aspect above;
III. a composition, comprising: a Cas protein, and a nucleic acid molecule 2 encoding the sgRNA described in the first aspect above;
IV. a composition, comprising: the nucleic acid molecule 1 and the nucleic acid molecule 2;
V. a composition, comprising: a nucleic acid molecule 3 encoding a Cas protein and the sgRNA described in the first aspect above;
VI. a composition, comprising: a Cas protein, and the sgRNA combination described in the second aspect above;
VII. a composition, comprising: the nucleic acid molecule 1, and the sgRNA combination described in the second aspect above;
VIII. a composition, comprising: a Cas protein and a nucleic acid molecule 4; the nucleic acid molecule 4 encoding both two sgRNAs in the sgRNA combination described in the second aspect above;
IX. a composition, comprising: a Cas protein, a nucleic acid molecule 5 and a nucleic acid molecule 6; the nucleic acid molecule 5 encoding any one sgRNA in the sgRNA combination described in the second aspect above; the expression vector 6 encoding the other sgRNA in the sgRNA combination in the second aspect above;
X. a composition, comprising: the nucleic acid molecule 1 and the nucleic acid molecule 4;
XI. a composition, comprising: the nucleic acid molecule 1, the nucleic acid molecule 5, and the nucleic acid molecule 6;
XII. a composition, comprising: a nucleic acid molecule 7 encoding a Cas protein and both two sgRNAs in the sgRNA combination described in the second aspect above;
XIII. a composition, comprising: a nucleic acid molecule 8 and a nucleic acid molecule 9; the nucleic acid molecule 8 encoding a Cas protein and any one sgRNA in the sgRNA combination described in the second aspect above; the nucleic acid molecule 9 encoding the other sgRNA in the sgRNA combination in the second aspect above.

In some examples, the sgRNA may bind to or interact with the Cas protein. That is, the sgRNA binds to or interacts with the Cas protein through the framework sequence of the sgRNA.

In some examples, the sgRNA may form a complex with the Cas protein.

In some examples, the sgRNA may guide the Cas protein to the target nucleic acid Aqpl mRNA. That is, the Cas13 nuclease is guided to a target sequence of the target nucleic acid Aqpl mRNA through complementation (complete complementation or partial complementation) between the targeting domain of the sgRNA and the target nucleic acid Aqp1 mRNA sequence.

In some examples, the sgRNA may guide Cas protein to the target nucleic acid Aqpl mRNA, and target or modify the target nucleic acid Aqp1 mRNA.

In some examples, the Cas protein is Cas13 protein.

In some examples, the Cas protein is Cas13a, Cas13b, Cas13c, Cas13d, Cas13e or Cas13f.

In some examples, the composition is selected from any one of the following:
I. a composition, comprising: a Cas13 protein, and the sgRNA of which the sequence of the targeting domain described in the first aspect above is SEQ ID NO: 5, SEQ ID NO: 21, SEQ ID NO: 24 or SEQ ID NO: 31;
II. a composition, comprising: a nucleic acid molecule 1 encoding a Cas13 protein, and the sgRNA of which the sequence of the targeting domain described above is SEQ ID NO: 5, SEQ ID NO: 21, SEQ ID NO: 24 or SEQ ID NO: 31;
III. a composition, comprising: a Cas13 protein, and a nucleic acid molecule 2 encoding the sgRNA of which the sequence of the targeting domain described in the first aspect above is SEQ ID NO: 5, SEQ ID NO: 21, SEQ ID NO: 24 or SEQ ID NO: 31;
IV. a composition, comprising: the nucleic acid molecule 1 and the nucleic acid molecule 2;
V. a composition, comprising: a nucleic acid molecule 3 encoding a Cas13 protein and the sgRNA of which the sequence of the targeting domain described in the first aspect above is SEQ ID NO: 5, SEQ ID NO: 21, SEQ ID NO: 24 or SEQ ID NO: 31;
the Cas13 protein can bind to or interact with the sgRNA.

In some examples, the nucleotide sequence encoding the sgRNA and/or the nucleotide sequence encoding the Cas protein in the nucleic acid molecule of the sixth aspect is operably linked to a promoter. The nucleic acid molecule may express sgRNA and/or Cas protein for targeting Aqp1 mRNA in cells (including but not limited to human cells).

The composition may be used to significantly down-regulate expression of Aqp1.

In a seventh aspect, the present disclosure claims a medicament.

The medicament claimed in the present disclosure comprises the sgRNA described in the first aspect above, or the sgRNA combination described in the second aspect above, or the DNA molecule described in the third aspect above, or the expression cassette, the expression vector, the recombinant bacterium, the recombinant virus or the transgenic cell line described in the fourth aspect above, or the composition described in the sixth aspect above, and the medicament comprises a pharmaceutically acceptable excipient.

In some examples, the medicament may be delivered using a delivery system including, but not limited to: RNP delivery, liposome delivery, nanoparticle delivery, virus delivery, and extracellular vesicle delivery.

In some examples of the present disclosure, virus delivery is used. In some examples, an AAV viral vector is used for delivery. Further, in some examples, AAV-ShH10 is used for delivery.

In an eighth aspect, the present disclosure claims a use of the sgRNA described in the first aspect above, or the sgRNA combination described in the second aspect above, or the DNA molecule described in the third aspect above, or the expression cassette, the expression vector, the recombinant bacterium, the recombinant virus or the transgenic cell line described in the fourth aspect above, or the composition described in the sixth aspect above, or the medicament described in the seventh aspect above in any one of the following:
Q1. preparation of a medicament for treating diseases in which downregulation of Aqpl expression is beneficial;
Q2. treatment of diseases in which downregulation of Aqpl expression is beneficial.

In some examples, the disease in which downregulation of Aqpl expression is beneficial is selected from tumors and glaucoma.

In some examples, the disease in which the downregulation of Aqp1 expression is beneficial is selected from glaucoma.

In a ninth aspect, the present disclosure claims a method for treatment of a disease in which downregulation of Aqpl expression is beneficial.

The method for the treatment of the disease in which downregulation of Aqp1 expression is beneficial claimed in the present disclosure is to treat the disease in which downregulation of Aqp1 expression is beneficial by using the sgRNA described in the first aspect above, or the sgRNA combination described in the second aspect above, or the DNA molecule described in the third aspect above, or the expression cassette, the expression vector, the recombinant bacterium, the recombinant virus or the transgenic cell line described in the fourth aspect above, or the composition described in the sixth aspect above, or the medicament described in the seventh aspect above.

Herein, the medicament may be delivered using a delivery system including, but not limited to: RNP delivery, liposome delivery, nanoparticle delivery, virus delivery, and extracellular vesicle delivery.

In some examples of the present disclosure, virus delivery is used. In some examples, an AAV vector is used for delivery. Further, in some examples, AAV-ShH10 is used for delivery.

In some examples, the disease in which downregulation of Aqp1 expression is beneficial is selected from tumors and glaucoma.

In some examples, the disease in which downregulation of Aqp1 expression is beneficial is selected from glaucoma.

### BRIEF DESCRIPTION OF THE DRAWINGS

FIG. 1 is a schematic diagram of sgRNA targeting target nucleic acid of the present disclosure.
FIG. 2 is a schematic diagram 1 of a target sequence of some sgRNAs on Aqpl mRNA of the present disclosure.
FIG. 3 is a schematic diagram 2 of a target sequence of some sgRNAs on Aqp1 mRNA of the present disclosure.
FIG. 4 is a schematic diagram 3 of a target sequence of some sgRNAs on Aqpl mRNA of the present disclosure.
FIG. 5 is a map of a RFZH-1 plasmid.
FIG. 6 exemplarily shows sequencing peak maps of some vectors of the present disclosure, wherein A-F correspond to sequencing peak maps of vectors of RFZH-1-Aqp1-sgRNA1, RFZH-1-Aqp1-sgRNA2, RFZH-1-Aqp1-sgRNA4, RFZH-1-Aqp1-sgRNA7, RFZH-1-Aqp1-sgRNA8, and RFZH-1-Aqp1-sgRNA9, respectively.
FIG. 7 is a map of a plasmid Lv-Aqp1 overexpressing Aqpl.

### DETAILED DESCRIPTION OF THE PREFERRED EMBODIMENT

### Definitions:

As used herein, a protein or polypeptide referred to as a "Cas protein", "Cas13 protein" or "Cas13 nuclease" relates to a CRISPR-associated (Cas) polypeptide or protein encoded by a CRISPR-associated (Cas) gene. The Cas protein or Cas13 protein can be guided to a target sequence in a target nucleic acid when being complexed or functionally combined with one or more guide RNAs (gRNAs). Under the guidance of the gRNA, Cas endonuclease recognizes, targets or modifies a specific target site (a target sequence or a nucleotide sequence near the target sequence) in the target nucleic acid, for example, a target site in an RNA (e.g., coding RNA, e.g., mRNA) molecule.

As used herein, "Cas13 protein" and "Cas13 nuclease" are used interchangeably. Cas13 proteins include natural Cas13 protein, artificially modified Cas13 protein, Cas13 mutant with nuclease activity loss or reduction, Cas 13 conjugate (non-limiting examples such as conjugate with nuclear localization signal NLS or nuclear export signal NES), fusion proteins of Cas13 (non-limiting examples such as a single base editor formed by fusion with deaminase).

As used herein, the terms "gRNA", "guide RNA", and "sgRNA" (single-molecule gRNA) are generally used interchangeably, and they have the meanings commonly understood by those skilled in the art. The gRNA generally refers to an RNA molecule (or a collective name of a group of RNA molecules) that can bind to or interact with a Cas protein or a Cas 13 protein and facilitate to guide/target the Cas protein or Cas13 protein to a specific position (target sequence) within a target nucleic acid/target polynucleotide (e.g., a DNA or mRNA molecule). The gRNA includes a guide sequence and a direct repeat (DR) sequence. The gRNA may contain one or more modifications (e.g., base modification, framework modification, modification of an internucleoside bond, etc.) to provide the same function as an unmodified gRNA, or to provide a new or enhanced feature (e.g., improved stability) to the gRNA. A suitable direct repeat (DR) sequence can be found from a CRISPR locus structure of a prokaryote (e.g., a bacterium and an archaebacterium) or obtained by experimental screening. The size of the direct repeat sequence is usually within tens of nt (nucleotides), and some of its fragments are complementary, which means that a secondary structure, such as a stem-loop structure (often called a hairpin structure), is formed inside the RNA molecule, while other fragments are embodied as unstructured. The direct repeat sequence is a constant part of a gRNA molecule, which contains a strong secondary structure, which facilitates the interaction between the Cas13 protein and the gRNA molecule.

As used herein, the terms "guide sequence" and "targeting domain" are used interchangeably, and refer to a continuous nucleotide sequence in a gRNA, which is partially or completely complementary to the target sequence in the target nucleic acid, and can hybridize with the target sequence in the target nucleic acid through base pairing promoted by the Cas protein or Cas13 protein. Complete complementarity between the guide sequence and the target sequence described in the present disclosure is not necessary, as long as there is sufficient complementarity to hybridize with the target sequence in the target nucleic acid and guide the CRISPR complex (complex of Cas protein and sgRNA) to specifically bind to the target sequence.

As used herein, the terms "target nucleic acid", "target RNA" or "target polynucleotide" refer to a polynucleotide containing a target sequence, and are often used interchangeably herein. The target nucleic acid may include any polynucleotide, such as a DNA (target DNA) or an RNA (target RNA). The "target nucleic acid" refers to a nucleic acid to be targeted or modified by Cas protein or the Cas13 protein as guided by the gRNA. The term "target nucleic acid" can be any polynucleotide endogenous or exogenous to a cell (e.g., an eukaryotic cell).

As used herein, the term "target sequence" refers to a small stretch of sequence in a target nucleic acid molecule, which can be complementary (completely or partially complementary) to or hybridized with the guide sequence of the gRNA molecule. The target sequence is often tens of bp in length, and for example, it can be about 10 bp, about 20 bp, about 30 bp, about 40 bp, about 50 bp and about 60 bp.

As used herein, when referring to targeting target nucleic acid Aqpl mRNA, the term "targeting" is defined to include one or more of the following:
cleaving Aqpl mRNA, visualizing or detecting Aqpl mRNA, labeling Aqpl mRNA, transporting Aqpl mRNA, masking Aqpl mRNA, increasing transcription and/or translation level of Aqpl mRNA, and reducing the transcription and/or translation level of Aqpl mRNA, altering splicing of pre-mRNA.

For example, in one example, the method of targeting a target RNA results in detecting, visualizing, or the target RNA (Aqpl mRNA). By using a Cas13 protein with a mutated HEPN domain, a guide RNA, and an effector module, the target RNA will be recognized by Cas13, but will not be cleaved or nicked, and the effector module will be activated. Such methods can be used in cellular or cell-free systems, for example, to determine the presence or level of expression of a target RNA in ocular cells. In one example, the Cas13 protein is fused to a fluorescent protein or other detectable marker, and the binding of Cas13 to the target RNA can be visualized by microscopy or other imaging methods.

In one example, targeting a target RNA allows for activation or increased expression of the target RNA. For example, fusing Cas13 with the mutated HEPN domain to a translational activation domain (e.g., eIF4E and other translation initiation factors), together with a guide RNA, can increase the expression of target RNA.

In one example, targeting a target RNA allows for inhibition or reduction of expression of the target RNA. For example, fusing Cas13 with the mutated HEPN domain to a translational repression domain (e.g., deadenylase), together with a guide RNA, can inhibit the expression of the target RNA.

As used herein, when referring to modifying the target nucleic acid Aqpl mRNA, the term "modifying" is defined to include one or more of the following:
base substitution of Aqpl mRNA, base deletion of Aqpl mRNA, base insertion of Aqpl mRNA, methylation of Aqpl mRNA, demethylation of Aqpl mRNA, and deamination of Aqp1 mRNA.

As used herein, "diseases in which downregulation of Aqpl expression is beneficial" is intended to refer to some diseases that can be prevented, treated, and the symptoms thereof can be relieved when Aqpl mRNA level or Aqpl protein level of specific organs, specific tissues or specific cells (intracellular, extracellular, or both intracellular and extracellular) is decreased or reduced in an organism. For example, glaucoma is treated by reducing the production of aqueous humor through functional loss of Aqp1 and lowering intraocular pressure.

As used herein, the term "operably linked" is intended to mean that a Cas protein coding sequence or Cas13 protein coding sequence or gRNA coding sequence in s vector is linked to one or more regulatory elements in a way that allows expression of the nucleotide sequence (e.g., expressing in an *in vitro* transcription/translation system or in a host cell when the vector is introduced into the host cell). The "regulatory element" is intended to include a promoter, an enhancer, an internal ribosome entry site (IRES), and other expression control elements (e.g., a transcription termination signal, such as a polyadenylation signal and a poly(U) sequence)

The following examples facilitate a better understanding of the present disclosure, but do not limit the present disclosure. The experimental method that does not indicate specific condition in the following examples is usually based on conventional conditions, such as conditions described in Sambrook et al., Molecular cloning: A Laboratory Manual (New York: Cold Spring Harbor Laboratory Press, 1989), or the conditions suggested by the manufacturer. Various commonly used chemical reagents used in the examples are all commercially available products.

### Example 1. Design and construction of sgRNA vector

sgRNA targeting Aqpl mRNA was designed. The sgRNA has a nucleic acid sequence including a targeting domain sequence and a framework sequence which are directly connected. The framework sequence is located at 5' end of the targeting domain sequence. The targeting domain sequence is reversely complementary to a target sequence on Aqp1 mRNA molecule. The targeting domain sequence is 20 to 30 nt in length. As shown in FIG. 1.

The framework sequence of the sgRNA in this example is 5'-CAAGUAAACCCCUACCAACUGGUC GGGGUUUGAAAC-3' (SEQ ID NO: 32).

The targeting domain sequences of sgRNAs designed by the inventors are shown in Table 1 below. FIGS. 2 to 4 show positions of target sequences corresponding to designed sgRNAs on human Aqp1 mRNA.

Herein, sgRNA1 to sgRNA31 were designed to target the CDS region of human Aqpl mRNA in plasmid transfection cell experiments through CRISPR-CasRx system.

**Table 1. Sequences of targeting domains of sgRNAs**

| sgRNANo. | Targeting domain sequence (5'→3') | Targeting domain sequence ID No. |
|---|---|---|
| sgRNA 1 | gcccuccagaagagcuucuucuuga | SEQ ID NO: 1 |
| sgRNA 2 | ccuccagaagagcuucuucuuga | SEQ ID NO: 2 |
| sgRNA 3 | caggaacucggccaccacugccc | SEQ ID NO: 3 |
| sgRNA 4 | aagacaaagagggucguggccag | SEQ ID NO: 4 |
| sgRNA 5 | agggcagaaccgaugcugaugaagac | SEQ ID NO: 5 |
| sgRNA 6 | caggaacucggccaccacugcc | SEQ ID NO: 6 |
| sgRNA 7 | auuugaagcccagggcagaaccg | SEQ ID NO: 7 |
| sgRNA 8 | ugguuguuccccaccggguauuug | SEQ ID NO: 8 |
| sgRNA 9 | ccgucugguuguuccccaccgggu | SEQ ID NO: 9 |
| sgRNA 10 | ccgccgucugguuguuccccacc | SEQ ID NO: 10 |
| sgRNA 11 | gcgccagcguggcgaugcucagcccgaagg | SEQ ID NO: 11 |
| sgRNA 12 | gccagcgacaccuucacguuguccuggacc | SEQ ID NO: 12 |
| sgRNA 13 | aagaugcugaucuggcagcugag | SEQ ID NO: 13 |
| sgRNA 14 | augauguacaugagggcacggaa | SEQ ID NO: 14 |
| sgRNA 15 | cgaguucacaccaucagccaggu | SEQ ID NO: 15 |
| sgRNA 16 | gcccaggcccuggcccgaguuca | SEQ ID NO: 16 |
| sgRNA 17 | ucccgaugaucucgaugcccagg | SEQ ID NO: 17 |
| sgRNA 18 | gccaguguagucaauagccagga | SEQ ID NO: 18 |
| sgRNA 19 | aucccacagccaguguagucaau | SEQ ID NO: 19 |
| sgRNA 20 | cugaaguugugugugaucaccgcgc | SEQ ID NO: 20 |
| sgRNA 21 | cccacccagaaaauccagugguu | SEQ ID NO: 21 |
| sgRNA 22 | cccagaaaauccagugguugcug | SEQ ID NO: 22 |
| sgRNA 23 | aagucguagaugaguacagccag | SEQ ID NO: 23 |
| sgRNA 24 | cugcugcguggggccaggaugaaguc | SEQ ID NO: 24 |
| sgRNA 25 | caccuucacgcggucugugaggucac | SEQ ID NO: 25 |
| sgRNA 26 | gcauccaggucauacuccuccaccuggcc | SEQ ID NO: 26 |
| sgRNA 27 | gucauacuccuccaccuggccgc | SEQ ID NO: 27 |
| sgRNA 28 | gucauacuccuccaccuggc | SEQ ID NO: 28 |
| sgRNA 29 | gcauccaggucauacuccuccac | SEQ ID NO: 29 |
| sgRNA 30 | cuucaucuccacccuggaguugaugucguc | SEQ ID NO: 30 |
| sgRNA 31 | uuugggcuucaucuccacccugga | SEQ ID NO: 31 |

Oligo DNAs corresponding to the target sequences (reverse complementary sequence of the targeting domain) were synthesized, respectively. According to the selection of the BpiI restriction site, AAAC was added to 5' end of the reverse complementary sequence of the target sequence, and CTTG was added to 5' end of the target sequence.

The sense strand of the Oligo DNA corresponding to the target sequence and the corresponding antisense strand were mixed, incubated at 95°C for 5 minutes, and placed on ice to cool and anneal for forming double-stranded DNA with sticky ends.

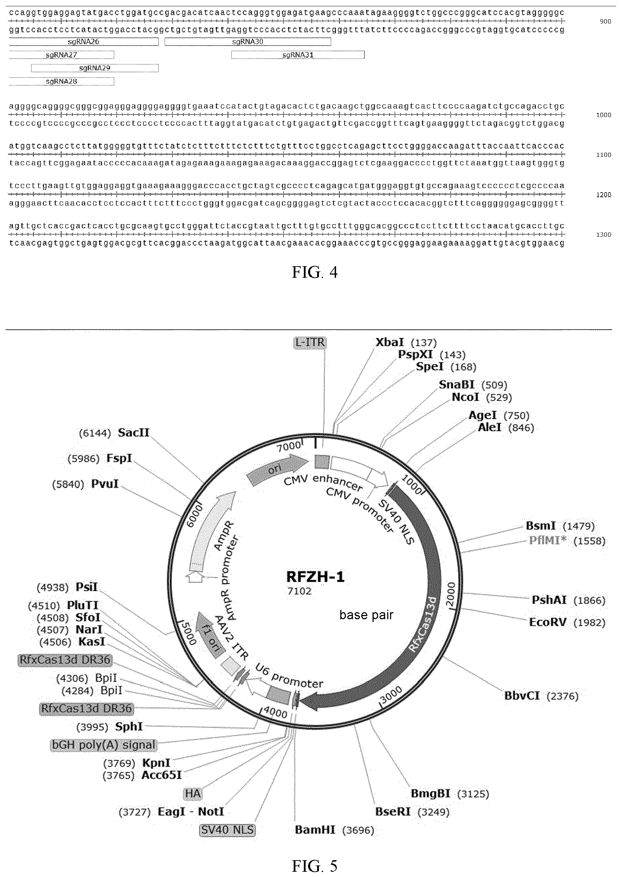

The map of RFZH-1 plasmid (SEQ ID NO: 33) is shown in FIG. 5, which was constructed by the inventors. This plasmid is used to construct CasRx and sgRNA expression vectors.

The RFZH-1 plasmid was linearized and digested with BpiI, and reacted at 37°C for 1 hour. The digested products were electrophoresed in 1% agarose gel, and the digested products were recovered by cutting the gel.

The annealed product and the recovery product linearized and digested by RFZH-1-BpiI were ligated using T4 ligase, and E.coli competent cells Stbl3 were transformed by the ligated product through heat shock method. After the transformation, LB liquid medium without antibiotics was added to a centrifuge tube. The centrifuge tube was placed in a constant-temperature shaker for shaking and culture at 37°C and 200 rpm to recover the bacteria.

The recovered Stbl3 cells were coated on ampicillin-resistant LB agar plates, and cultured upside down in a constant-temperature incubator at 37°C. Single colonies were picked from the above plates and inoculated into 50 mL liquid LB medium containing 50 µl ampicillin. Primers U6 Promoter-F (5'-GGGCCTATTTCCCATGATTCCTT-3', SEQ ID NO: 34) and flori-R (5 '-GCTGGCAAGTGTAGCGGTCA-3', SEQ ID NO: 35) were used to perform PCR identification on the above bacterial liquid. After the PCR products were subjected to 1% agarose gel electrophoresis, the bacterial liquid containing positive clones was screened out, inoculated into 5 mL of 50 mL LB liquid medium containing ampicillin, and cultured with shaking at 37°C and 200 rpm for 16 hours.

After the plasmids were extracted and the concentration of the plasmid was determined, some plasmids were taken for Sanger sequencing, and the correctly sequenced plasmids were stored at -20°C for future use.

The vector containing the sequence encoding sgRNAn targeting Aqpl mRNA was named as RFZH-1-Aqp1-sgRNAn (n is a numerical sequence number, and sgRNAn corresponds to the sgRNA number in Table 1).

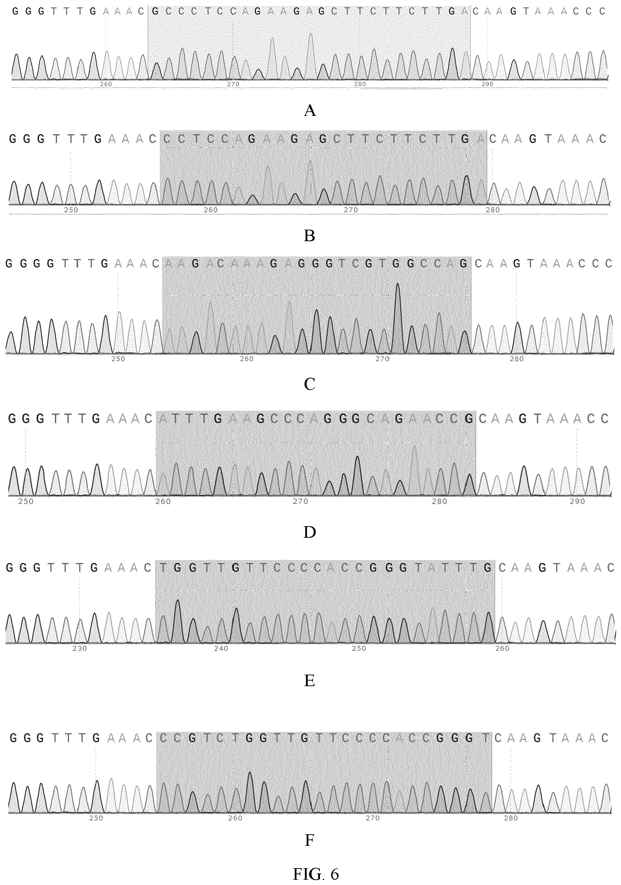

FIG. 6 exemplarily shows the sequencing peak maps of some vectors. Figures A-F correspond to the sequencing peak maps of vectors of RFZH-1-Aqp1-sgRNA1, RFZH-1-Aqp1-sgRNA2, RFZH-1-Aqp1-sgRNA4, RFZH-1-Aqp1-sgRNA7, RFZH-1-Aqp1-sgRNA8, and RFZH-1-Aqp1-sgRNA9, respectively.

### Example 2. Plasmid transfection and identification

Serum-free medium was used to dilute Lipofectamine 2000 liposome transfection reagent, and the plasmids RFZH-1-Aqp1-sgRNAn1, RFZH-1-Aqp1-sgRNAn2 (n1, n2 represent any two plasmids encoding sgRNA 1 to sgRNA 31 in Table 1) cloned with sgRNA constructed in Example 1 and the plasmid Lv-Aqp1 overexpressing Aqpl were co-transfected at a mass ratio of 12:12:1 (250 ng in total). Alternatively, only one sgRNA plasmid was transfected. The RFZH-1-Aqp1-sgRNAn1 (n1 represents any one plasmid encoding sgRNA 1 to sgRNA31 in Table 1) constructed in Example 1 and the Lv-Aqp1 plasmid overexpressing Aqpl were co-transfected into 293T cells at a mass ratio of 24:1 (250 ng in total).

RFZH-1 plasmid and Lv-Aqp1 plasmid were transfected in negative control group.

Only RFZH-1 plasmid was transfected in blank control group.

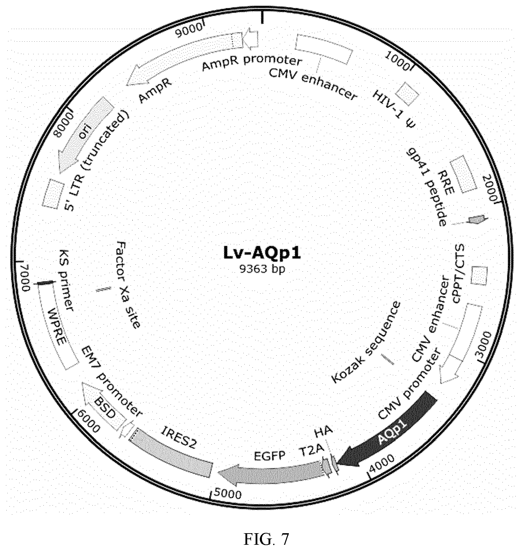

The plasmid Lv-Aqp1 (SEQ ID NO: 36) overexpressing Aqpl was constructed by the inventors, and its map is shown in FIG. 7.

After mixing the two plasmid dilutions and keeping the temperature for 20 minutes, the complex was added to a 24-well plate, and then the cell suspension was added to the complex.

After 72 hours of transfection with the plasmid, the total RNA of 293T cells was extracted using universal RNA extraction kits from Accurate Biotech, and the corresponding cDNA was obtained by using the Evo M-MLV reverse transcription kit for gDNA digestion and reverse transcription. SYSB Green Pro Taq HS premixed qPCR kit was used in the Q-PCR reaction. GAPDH was used as an internal reference, and Roche lightcycler 480II was used to obtain the Aqp1 mRNA level after target editing in each group. Q-PCR was done with 3 replicate wells in parallel for each sample and each pair of primers. Q-PCR primers are shown in Table 2.

**Table 2. Primers used in 293T cells for Q-PCR**

| Primer name | Sequence 5'→3' | Sequence ID No. |
|---|---|---|
| Q-hGAPDH-F | CCATGGGGAAGGTGAAGGTC | SEQ ID NO: 37 |
| Q-hGAPDH-R | GAAGGGGTCATTGATGGCAAC | SEQ ID NO: 38 |
| hAqp1-F | gctcttctggagggcagtgg | SEQ ID NO: 39 |
| hAqp1-R | cagtgtgacagccgggttgag | SEQ ID NO: 40 |

### Example 3. Detection result of Aqpl mRNA expression

Transfection of cells with recombinant plasmid, extraction of RNA, synthesis of cDNA by reverse transcription, and qPCR was carried out in three independent biological repeat experiments, and the average results of the three experiments were obtained by calculation method of 2^-ΔΔCt.

The test results of the Aqpl mRNA expression are shown in Table 3 below. The result data was represented by the average value of the results of the three experiments. The Aqpl mRNA level of the blank control group was counted as 0, and the Aqpl mRNA level of the negative control group was counted as 1.

**Table 3. Aqp1 mRNA levels after editing 293T cells by CRISPR system**

| Group | Relative expression of Aqp1 mRNA |
|---|---|
| Blank control | 0.00 |
| Negative control | 1.00 |
| sgRNA 1 | 0.11* |
| sgRNA 2 | 0.12* |
| sgRNA 3 | 0.77* |
| sgRNA 4 | 0.14* |
| sgRNA 5 | 0.17* |
| sgRNA 6 | 0.69* |
| sgRNA 7 | 0.16* |
| sgRNA 8 | 0.25* |
| sgRNA 9 | 0.38* |
| sgRNA 10 | 0.28* |
| sgRNA 11 | 0.76* |
| sgRNA 12 | 0.21* |
| sgRNA 13 | 0.55* |
| sgRNA 14 | 0.21* |
| sgRNA 15 | 0.22* |
| sgRNA 16 | 0.85 * |
| sgRNA 17 | 0.17* |
| sgRNA 18 | 0.16* |
| sgRNA 19 | 0.71* |
| sgRNA 20 | 0.15* |
| sgRNA 21 | 0.16* |
| sgRNA 22 | 0.73* |
| sgRNA 23 | 0.22* |
| sgRNA 24 | 0.16* |
| sgRNA 25 | 0.64* |
| sgRNA 26 | 0.23* |
| sgRNA 27 | 0.67* |
| sgRNA 28 | 0.18* |
| sgRNA 29 | 0.64* |
| sgRNA 30 | 0.21* |
| sgRNA 31 | 0.17* |
| sgRNA 2 + sgRNA 5 | 0.03* |
| sgRNA 2 + sgRNA 21 | 0.07* |
| sgRNA 2 + sgRNA 24 | 0.03* |
| sgRNA 2 + sgRNA 31 | 0.06* |
| sgRNA 5 + sgRNA 21 | 0.04* |
| sgRNA 5 + sgRNA 24 | 0.05* |
| sgRNA 5 + sgRNA 31 | 0.03* |
| sgRNA 21 + sgRNA 24 | 0.09* |
| sgRNA 21 + sgRNA 31 | 0.09* |
| sgRNA 24 + sgRNA 31 | 0.07* |

| | |
|---|---|
| Note: * means there is a statistical difference (P<0.05) compared with the negative control group. | |

The data in Table 3 shows that the inventors significantly reduced the expression level of Aqpl mRNA through sgRNA-Cas13 system.

In human 293T cells, sgRNA 1-2, 4-5, 7-10, 12, 14-15, 17-18, 20-21, 23-24, 26, 28, 30-31 had the strongest knockdown effect on Aqpl mRNA, followed by sgRNA 3, 6, 11, 13, 16, 19, 22, 25, 27, 29.

In summary, the inventors successfully constructed a CRISPR-Cas13 system targeting Aqpl mRNA, which can effectively reduce the level of Aqpl mRNA.

Targeting Aqpl mRNA by CRISPR-Cas13 is safer than targeting Aqpl DNA directly by CRISPR-Cas.

### Example 4: In vivo efficacy test in animals

RFZH-1-Aqp1-sgRNA C plasmid (for subsequent positive control) and RFZH-1-Aqpl-sgRNA 21 plasmid (targeting mouse Aqpl mRNA) which could express CasRx and sgRNA C (targeting C57BL/6J mouse Aqpl mRNA) were constructed by using the method in Example 1. The targeting domain sequence of sgRNA C was 5'-ACUUUGGGCCAGAGUAGCGAU-3' (SEQ ID NO: 41).

The conventional triple-transfection method was used in the reagent company. RFZH-1-Aqp1-sgRNA 21 plasmid or RFZH-1-Aqp1-sgRNA C plasmid, shh10 plasmid (addgene Plasmid #64867), and auxiliary plasmid pAdDeltaF6 (addgene Plasmid #112867) were transfected, cultured in 293T cells and separated to obtain serotype AAV-ShH10 (which could express CasRx driven by CMV in eukaryotic cells, and express sgRNA 21 or sgRNA C driven by U6). The sequence packaged in the capsid of AAV -ShH10 was ITR in the RFZH-1-Aqp1-sgRNA21 plasmid or RFZH-1-Aqp1-sgRNAC plasmid and the sequence between the RFZH-1-Aqp1-sgRNA21 plasmid and RFZH-1-Aqp1-sgRNA C plasmid.

According to the above triple-transfection method, the gRNA empty plasmid (RFZH-1), shh10 plasmid (addgene Plasmid #64867), and auxiliary plasmid pAdDeltaF6 (addgene Plasmid #112867) were transfected into 293T cells, cultured and separated to obtain AAV-ShH10 of sgRNA empty (which expressed CasRx driven by CMV; and the gRNA driven by U6 targeted the RNA sequence of Takifugu rubripes instead of targeting Aqp1).

In addition, using the above triple-transfection method, GFP plasmid (SEQ ID NO: 42), shh10 plasmid (addgene Plasmid #64867), and auxiliary plasmid pAdDeltaF6 (addgene Plasmid #112867) were transfected into 293T cells, cultured and separated to obtain AAV-ShH10 expressing GFP (CMV-driven expression), referred to as GFP-AAV or ShH10-GFP.

Ten normal 6-week-old male C57BL/6J mice with stable intraocular pressure were selected, wherein 5 mice were injected with AAV (2×10⁹ GC AAV) and GFP AAV (2×10⁸ GC AAV) expressing sgRNA 21 in the vitreous humor of their left eyes. Another 5 mice were injected with AAV (2×10⁹ GC AAV) and GFP AAV (2×10⁸ GC AAV) expressing sgRNA C in the vitreous humor of their left eyes. All 10 mice were injected with sgRNA empty AAV (2×10⁹ GC AAV) and GFP AAV (2×10⁸ GC AAV) in the vitreous humor of their right eyes. Herein, GC = Genome copies. As shown in Table 4 below.

**Table 4. Grouping of animal experiments**

| Group | Administration |
|---|---|
| Negative control | sgRNA empty AAV + GFP AAV |
| Test Group | sgRNA 21 AAV + GFP AAV |
| Positive control | sgRNA C AAV+ GFP AAV |

One week after injection, the coverage of GFP in ciliary body was detected. The distribution of GFP was tested by fluorescence, which showed that the drug injection was successful. Three weeks after the injection, Icare^{®} TONOLAB tonometer was used to measure the intraocular pressure of both eyes. The intraocular pressure of any one eye of each mouse was tested 6 times continuously. The results are shown in Table 5 and Table 6 below.

**Table 5. Intraocular pressure test results of mice injected with sgRNA21 AAV for 3 weeks**

| | Intraocular pressure (mm Hg) after sgRNA 21 AAV injection in the left eye | Intraocular pressure (mm Hg) after sgRNA empty AAV injection in the right eye |
|---|---|---|
| Average intraocular pressure of 5 mice | 10.5* | 14.5 |

| | | |
|---|---|---|
| Note: * indicates that the mean value of the experimental group is statistically different (P<0.05) from the mean value of the negative control group. | | |

**Table 6. Intraocular pressure test results of mice injected with sgRNA C AAV for 3 weeks**

| | Intraocular pressure (mm Hg) after sgRNA C AAV injection in the left eye | Intraocular pressure (mm Hg) after sgRNA empty AAV inj ection in the right eye |
|---|---|---|
| Average intraocular pressure of 5 mice | 12.9* | 14.1 |

| | | |
|---|---|---|
| Note: * indicates that the mean value of the experimental group is statistically different (P<0.05) from the mean value of the negative control group. | | |

As shown in Table 5, sgRNA21 can effectively reduce the intraocular pressure of mice compared with the empty. Table 6 shows that sgRNA C can effectively reduce the intraocular pressure of mice compared with the empty. Therefore, sgRNA 21 and sgRNA C can be used for the treatment of glaucoma.

In terms of the reduction of intraocular pressure, sgRNA 21 of the present disclosure has a better effect than sgRNA C.

### Example 5. Off-target detection

1. EMBOSS-water program and NCBI-Blast program were used to predict the whole genome and full cDNA sequence of the target species (ICR mouse), and the sense strand and antisense strand of the sgRNA5 and sgRNA31 guide sequences in Table 1 were respectively used to compare the prediction results. The prediction results were filtered according to the requirements of the difference between the length of the predicted target and length of the sgRNA guide sequence not exceeding four bases, and mismatch+gap not exceeding four bases. The potential off-target information was obtained as follows:
   sgRNA5: 125 potential off-target genes; 255 potential off-target transcripts.
   sgRNA31: 1285 potential off-target genes; 2496 potential off-target transcripts.
2. Vitreous administration of male ICR mice, AAV ShH10 was administered to both eyes.

For an AAV ShH10 which can express CasRx, sgRNA5, and sgRNA31, a preparation method thereof is as follows: using the conventional triple-transfection method, that is, by transfecting RFZH-1-Aqp1-sgRNA5+31 plasmid (SEQ ID NO: 43), shh10 plasmid (addgene Plasmid #64867), and the auxiliary plasmid pAdDeltaF6 (addgene Plasmid #112867), culturing and separating in 293T cells to obtain serotype AAV-ShH10, which can express CasRx driven by CMV and express sgRNA5 and sgRNA31 driven by U6, and is named ShH10-CRISPR.

For another AAV ShH10 which can express GFP, a preparation method thereof is as follows: by using the conventional triple-transfection method, that is, by transfecting GFP plasmid (SEQ ID NO: 42), shh10 plasmid (addgene Plasmid #64867), and the auxiliary plasmid pAdDeltaF6 (addgene Plasmid #112867), culturing and separating in 293T cells to obtain AAV-ShH10, which can express GFP (CMV-driven expression), and is called ShH10-GFP.

Six mice in the experimental group were administered ShH10-CRISPR (titer: 1E+13 GC/ml) and ShH10-GFP (titer: 1E+13 GC/ml) in total 2 µL (volume ratio 10:1) per eye at the same time, and six mice of the control group were administered 2 µL of ShH10-GFP per eye (titer: 1E+13 GC/ml). After 27 days, a complete retinal sample was taken. The total RNA of the sample was extracted by the phenol-chloroform method, and PE150bp RNA-Seq sequencing was performed. The fastq file obtained by sequencing was compared with the reference genome of the target species (ICR mouse) by HISAT2 and STAR software to obtain the compared BAM file. The transcripts and the expression levels of each gene were tested by kallisto, RSEM and HTSeq.

3. DESeq2, limma-voom, and edger were used to analyze the differences in the expression levels between the groups. The genes satisfying p.adj<0.05, Ilog2FoldChangel>=0.75, basemean>2.5 were regarded as differentially expressed genes (DEGs), and a total of DEG information was as follows:
73 DEGs were significantly upregulated; 80 transcripts were significantly down-regulated.
39 DEGs were significantly down-regulated; 42 transcripts were significantly down-regulated.

4. The intersection of the significantly down-regulated genes among the differentially expressed genes and the predicted potential off-target genes was taken as an off-target gene with high reliability. Related information was as follows:
0 sgRNA5-related off-target genes; 0 related transcripts.
6 sgRNA31-related off-target genes; 6 related transcripts.

Therefore, in terms of off-target editing, the safety of sgRNA5 is superior to that of sgRNA31.

The above examples are preferred embodiments of the present disclosure, but the embodiments of the present disclosure are not limited by the above examples, and any other changes, modifications, substitutions, combinations, and simplifications should be equivalent replacement methods, and all are included within the protection scope of the present disclosure.

### Industrial application

The present disclosure uses the CRISPR/Cas system for the first time, provides sgRNA targeting human Aqpl mRNA sequence, and achieves significant downregulation of Aqpl mRNA level. After being delivered to cells by a vector, the sgRNA targets Aqp1 mRNA and reduces the level of Aqpl mRNA. The sgRNA can be used to treat diseases in which downregulation of Aqp1 expression is beneficial, including but not limited to diseases related to Aqpl overexpression (including cancer, glaucoma), or treat related diseases by downregulating the conventional expression level of Aqp1. The sgRNA targeting the mRNA sequence of the Aqp1 gene provided in the present disclosure is safer than directly targeting the Aqp1 gene. The sgRNA of the present disclosure can effectively reduce the Aqp1 mRNA level. Through design, the inventors found sgRNAs with significantly better effects near some sgRNA target sites, which can more effectively reduce the Aqp1 mRNA level.

## Claims

1. An sgRNA, wherein the sgRNA can target human Aqpl mRNA.

2. The sgRNA of claim 1, wherein the sgRNA has a nucleotide sequence comprising a targeting domain and a framework sequence, and the targeting domain is complementary to a target nucleic acid Aqpl mRNA sequence, and the framework sequence can interact with a Cas protein.

3. The sgRNA of claim 2, wherein the Cas protein is a Cas13 protein.

4. The sgRNA of any one of claims 1 to 3, wherein the targeting domain comprises any one of sequences shown in SEQ ID NO: 1 to SEQ ID NO: 31.

5. The sgRNA of claim 4, wherein the targeting domain of the sgRNA comprises any one of sequences shown in SEQ ID NO: 1, SEQ ID NO: 2, SEQ ID NO: 4, SEQ ID NO: 5, SEQ ID NO: 7, SEQ ID No: 8, SEQ ID NO: 9, SEQ ID NO: 10, SEQ ID NO: 12, SEQ ID NO: 14, SEQ ID NO: 15, SEQ ID NO: 17, SEQ ID NO: 18, SEQ ID NO: 20, SEQ ID NO: 21, SEQ ID NO: 23, SEQ ID NO: 24, SEQ ID NO: 26, SEQ ID NO: 28, SEQ ID NO: 30, and SEQ ID NO: 31.

6. The sgRNA of claim 5, wherein the targeting domain of the sgRNA comprises any one of sequences shown in SEQ ID NO: 2, SEQ ID NO: 5, SEQ ID NO: 21, SEQ ID NO: 24, and SEQ ID NO: 31.

7. An sgRNA combination composed of any two different sgRNAs from any one of claims 1 to 6.

8. The sgRNA combination of claim 7, wherein the sgRNA combination is selected from any one of the following:
P1. the sgRNA of which the targeting domain has a sequence of SEQ ID NO: 2, and the sgRNA of which the targeting domain has a sequence of SEQ ID NO: 5;
P2. the sgRNA of which the targeting domain has a sequence of SEQ ID NO: 2, and the sgRNA of which the targeting domain has a sequence of SEQ ID NO: 21;
P3. the sgRNA of which the targeting domain has a sequence of SEQ ID NO: 2, and the sgRNA of which the targeting domain has a sequence of SEQ ID NO: 24;
P4. the sgRNA of which the targeting domain has a sequence of SEQ ID NO: 2, and the sgRNA of which the targeting domain has a sequence of SEQ ID NO: 31;
P5. the sgRNA of which the targeting domain has a sequence of SEQ ID NO: 5, and the sgRNA of which the targeting domain has a sequence of SEQ ID NO: 21;
P6. the sgRNA of which the targeting domain has a sequence of SEQ ID NO: 5, and the sgRNA of which the targeting domain has a sequence of SEQ ID NO: 24;
P7. the sgRNA of which the targeting domain has a sequence of SEQ ID NO: 5, and the sgRNA of which the targeting domain has a sequence of SEQ ID NO: 31;
P8. the sgRNA of which the targeting domain has a sequence of SEQ ID NO: 21, and the sgRNA of which the targeting domain has a sequence of SEQ ID NO: 24;
P9. the sgRNA of which the targeting domain has a sequence of SEQ ID NO: 21, and the sgRNA of which the targeting domain has a sequence of SEQ ID NO: 31;
P10. the sgRNA of which the targeting domain has a sequence of SEQ ID NO: 24, and the sgRNA of which the targeting domain has a sequence of SEQ ID NO: 31.

9. A DNA molecule encoding the sgRNA of any one of claims 1 to 6 or the sgRNA combination of claim 7 or 8.

10. An expression cassette, an expression vector, a recombinant bacterium, a recombinant virus or a transgenic cell line containing the DNA molecule of claim 9.

11. A kit, comprising any one of the following:
i. a Cas protein, and the sgRNA of any one of claims 1 to 6;
ii. an expression vector 1 comprising a nucleotide sequence encoding a Cas protein, and the sgRNA of any one of claims 1 to 6;
iii. a Cas protein, and an expression vector 2 comprising a nucleotide sequence encoding the sgRNA of any one of claims 1 to 6;
iv. the expression vector 1 and the expression vector 2;
v. an expression vector 3 comprising a nucleotide sequence encoding a Cas protein and a nucleotide sequence encoding the sgRNA of any one of claims 1 to 6;
vi. a Cas protein, and the sgRNA combination of claim 7 or 8;
vii. the expression vector 1, and the sgRNA combination of claim 7 or 8;
viii. a Cas protein and an expression vector 4; the expression vector 4 comprising a nucleotide sequence encoding both two sgRNAs in the sgRNA combination of claim 7 or 8;
ix. a Cas protein, an expression vector 5 and an expression vector 6; the expression vector 5 comprising a nucleotide sequence encoding any one sgRNA in the sgRNA combination of claim 7 or 8; the expression vector 6 comprising a nucleotide sequence encoding the other sgRNA in the sgRNA combination of claim 7 or 8;
x. the expression vector 1 and the expression vector 4;
xi. the expression vector 1, the expression vector 5 and the expression vector 6;
xii. an expression vector 7 comprising a nucleotide sequence encoding a Cas protein and a nucleotide sequence encoding both two sgRNAs in the sgRNA combination of claim 7 or 8;
xiii. an expression vector 8 and an expression vector 9; the expression vector 8 comprising a nucleotide sequence encoding a Cas protein and a nucleotide sequence encoding any one sgRNA in the sgRNA combination of claim 7 or 8; the expression vector 9 comprising a nucleotide sequence encoding the other sgRNA in the sgRNA combination of claim 7 or 8;
the Cas protein can interact with the sgRNA.

12. The kit of claim 11, wherein the Cas protein is a Cas13 protein.

13. A composition, selected from any one of the following:
I. a composition, comprising: a Cas protein, and the sgRNA of any one of claims 1 to 6;
II. a composition, comprising: a nucleic acid molecule 1 encoding a Cas protein, and the sgRNA of any one of claims 1 to 6;
III. a composition, comprising: a Cas protein, and a nucleic acid molecule 2 encoding the sgRNA of any one of claims 1 to 6;
IV. a composition, comprising: the nucleic acid molecule 1 and the nucleic acid molecule 2;
V. a composition, comprising: a nucleic acid molecule 3 encoding a Cas protein and the sgRNA of any one of claims 1 to 6;
VI. a composition, comprising: a Cas protein, and the sgRNA combination of claim 7 or 8;
VII. a composition, comprising: the nucleic acid molecule 1, and the sgRNA combination of claim 7 or 8;
VIII. a composition, comprising: a Cas protein and a nucleic acid molecule 4; the nucleic acid molecule 4 encoding both two sgRNAs in the sgRNA combination of claim 7 or 8;
IX. a composition, comprising: a Cas protein, a nucleic acid molecule 5 and a nucleic acid molecule 6; the nucleic acid molecule 5 encoding any one sgRNA in the sgRNA combination of claim 7 or 8; the expression vector 6 encoding the other sgRNA in the sgRNA combination of claim 7 or 8;
X. a composition, comprising: the nucleic acid molecule 1 and the nucleic acid molecule 4;
XI. a composition, comprising: the nucleic acid molecule 1, the nucleic acid molecule 5, and the nucleic acid molecule 6;
XII. a composition, comprising: a nucleic acid molecule 7 encoding a Cas protein and both two sgRNAs in the sgRNA combination of claim 7 or 8;
XIII. a composition, comprising: a nucleic acid molecule 8 and a nucleic acid molecule 9; the nucleic acid molecule 8 encoding a Cas protein and any one sgRNA in the sgRNA combination of claim 7 or 8; the nucleic acid molecule 9 encoding the other sgRNA in the sgRNA combination of claim 7 or 8;
the Cas protein can interact with the sgRNA.

14. The composition of claim 13, wherein the Cas protein is a Cas13 protein.

15. A medicament, wherein the medicament comprises the sgRNA of any one of claims 1 to 6, or the sgRNA combination of claim 7 or 8, or the DNA molecule of claim 9, or the expression cassette, the expression vector, the recombinant bacterium, the recombinant virus or the transgenic cell line of claim 10, or the composition of claim 13 or 14, and the medicament comprises a pharmaceutically acceptable excipient.

16. The medicament of claim 15, wherein the medicament is delivered by following delivery systems: RNP delivery, liposome delivery, nanoparticle delivery, virus delivery or extracellular vesicle delivery.

17. A use of the sgRNA of any one of claims 1 to 6, or the sgRNA combination of claim 7 or 8, or the DNA molecule of claim 9, or the expression cassette, the expression vector, the recombinant bacterium, the recombinant virus or the transgenic cell line of claim 10, or the composition of claim 13 or 14, or the medicament of claim 15 in any one of the following:
Q1. preparation of a medicament for treating diseases in which downregulation of Aqp1 expression is beneficial;
Q2. treatment of diseases in which downregulation of Aqp1 expression is beneficial.

18. The use of claim 17, wherein the disease in which downregulation of Aqp1 expression is beneficial is selected from tumors and glaucoma.

19. A method for treatment of a disease in which downregulation of Aqp1 expression is beneficial is to treat the disease in which downregulation of Aqp1 expression is beneficial by using the sgRNA of any one of claims 1 to 6, or the sgRNA combination of claim 7 or 8, or the DNA molecule of claim 9, or the expression cassette, the expression vector, the recombinant bacterium, the recombinant virus or the transgenic cell line of claim 10, or the composition of claim 13 or 14, or the medicament of claim 15.

20. The method of claim 19, wherein the disease in which downregulation of Aqpl expression is beneficial is selected from tumors and glaucoma.
